# EUROPEAN PATENT SPECIFICATION

(11) **EP 1 480 558 B1**
(45) Date of publication and mention of the grant of the patent: **13.09.2006**
(21) Application number: 03706725.3
(22) Date of filing: 19.02.2003
(51) Int. Cl.: A61B 5/103

(54) **SKIN TESTING APPARATUS**
VORRICHTUNG ZUM HAUTTEST
APPAREIL DE TEST CUTANE

(30) Priority: 26.02.2002 GB 0204423; 07.03.2002 GB 0205338
(43) Date of publication of application: 01.12.2004
(73) Proprietor: HH Impress Limited, Liphook, Hampshire GU30 7ED (GB)
(72) Inventor: HULME, Alan, Greenock PA16 8HU (GB)
(74) Representative: McKechnie, Neil Henry
(86) International application number: PCT/GB2003/000759
(87) International publication number: WO 2003/071947

(56) References cited:
- WO-A-00/74558
- WO-A-92/14402
- GB-A- 2 182 458
- US-A- 5 369 527

## Description

The present invention relates to apparatus for testing a rash present on human skin in order to aid the diagnosis of a disease. In particular, the apparatus may be employed to quickly diagnose the presence of meningitis or septicaemia.

Meningitis is an inflammation of the tissues that cover the brain and spinal cord. It is a rare but very serious illness, however if diagnosed at an early stage most patients make a full recovery. Either bacteria or viruses cause the majority of cases in the UK. There are two main types of bacterial meningitis in the UK, named after the germs that cause the infection namely, meningococcal or pneumococcoal. However, there are other types including Hib and Group B strepococcal.

In its initial stages meningitis is difficult to identify as the symptoms are similar to those of influenza. In addition the symptoms may not all appear at the same time. However, characteristic symptoms include a red or purpuric rash, stiffness of the neck, drowsiness or confusion, severe headaches, vomiting, a temperature or an aversion to bright light.

Septicaemia is a form of blood poisoning that may be caused by the same germs that cause meningitis and in fact often occurs with meningococcal meningitis. Septicaemia is very serious and must be treated straight away. It is the more life-threatening consequence of the meningococcal infection.

With septicaemia the bacteria releases toxins into the blood which act to break down the walls of the blood vessels allowing blood to leak out under the skin. This leaking causes marks on the skin in the form of a rash of red or brownish pin prick spots which thereafter develop into purple bruises, blood blisters or blood spots. Such a rash can appear anywhere on the body. Septicaemia also makes the sufferer very ill as it reduces the amount of blood reaching the vital organs such as the liver and kidneys.

Anyone, of any age, can get meningitis or septicaemia however it is most common in teenagers and young children. At any one time it is estimated that in the UK one person in ten will be a carrier of the meningococcal bacteria. The bacteria are carried in the back of the nose and throat but in the majority of cases the carrier is unaware of its presence. However, in a few cases the bacteria overcomes the body's immune defences so passing through the lining of the nose and throat and into the blood stream. Annually in the UK there are an estimated 3,500 reported cases of meningococcal meningitis and septicaemia.

The reason why some people develop meningococcal meningitis or septicaemia from the meningococcal bacteria, while it remains harmless to the majority of carriers, is not yet fully understood. Both can be treated, however because of the rapid rate at which they develop it is of utmost importance for a sufferer to obtain medical assistance as soon as possible.

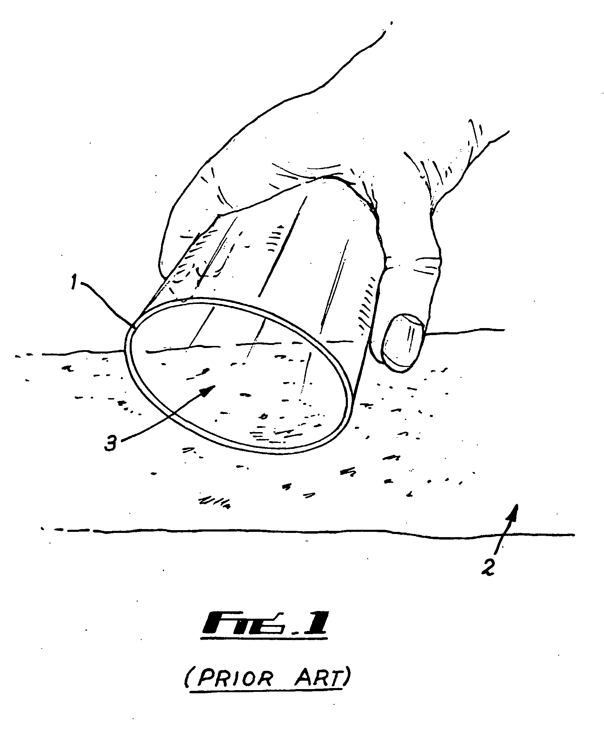

A recommended method for testing for meningococcal meningitis or septicaemia comprises the pressing of a glass tumbler 1 firmly against the skin 2, see Figure 1. By moving the glass tumbler 1 from side to side a person can check to see if the rash 3 blanches or loses its colour under pressure. If the rash 3 does not fade or lose colour the person is advised to immediately seek medical attention.

Document GB-A-2 182 458 discloses a device according to the preamble of claim 1. Meningococcal meningitis or septicaemia are not however the only diseases that produce a purpuric rash or a rash that does not blanch under pressure. For example Henoch-Schonlein Purpura is an inflammatory disorder that, as the name suggests, is characterised by a purpuric rash. Although still requiring medical attention it does not require the same speed of response as with either meningococcal meningitis or septicaemia.

The existence of other diseases that produce a purpuric rash or a rash that does not blanch under pressure highlights a flaw in employing a glass tumbler for the recommended method of testing for meningococcal meningitis or septicaemia. As the occurrence of both meningococcal meningitis and septicaemia are rare the exact form of their associated rashes is not well known to the general public. It requires a skilled eye to identify these associated rashes. This often results in the general public mistakenly diagnosing either meningococcal meningitis or septicaemia when in fact the cause of the purpuric rashes is in fact something quite different. This results in undue anxiety and stress on the part of the general public particularly when such rashes appear on the skin of young children.

It is an object of the present invention to provide apparatus for testing a rash present on human skin that aids the diagnosis of a diseases that is characterised by a particular skin rash.

According to the present invention there is provided an apparatus as defined in claim 1.

Optionally the reference section comprises a receiving means and a removable cartridge.

Preferably the cartridge comprises one or more replications of rashes associated with diseases to be tested for.

Preferably the replication of the rash comprises a representation of meningococcal meningitis. Alternatively the replication of the rash comprises a representation of septicaemia although any other rash associated with the human skin may be employed.

Optionally the apparatus further comprises one or more handles.

Example embodiments of the present invention will now be described with reference to the following figures:
Figure 1 shows a glass tumbler being employed for a pressure test as described in the Prior Art;
Figure 2 shows:
   (a) a side profile, and
   (b) a top profile,
   of apparatus for testing and diagnosing a rash present on human skin in accordance with the present invention;
Figure 3 shows:
   (a) a side profile, and
   (b) a top profile,
   of an alternative embodiment of the testing apparatus in accordance with the present invention; and
Figure 4 shows:
   (a) a side profile, and
   (b) a top profile,
   of an alternative embodiment of the testing apparatus, with a reference cartridge partially inserted, in accordance with the present invention.

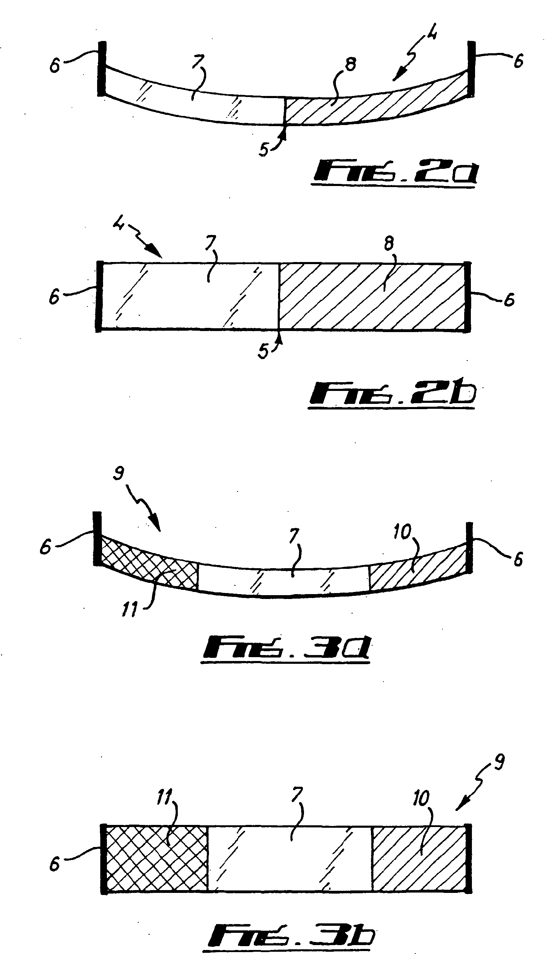

Referring to Figure 2 testing apparatus 4 for testing and diagnosing a rash present on human skin can be seen to comprise of a main body 5 and two handles 6. The main body 5 can be see to take the shape of a concave slide and itself comprises a transparent viewing section 7 and a reference section 8.

The reference section 8 provides a representation of a rash associated with a particular disease that is to be tested for. For example the reference section 8 may provide a representation of the rash associated with either meningococcal meningitis or septicaemia.

The testing apparatus 4 can be employed in a similar manner as described above for the glass tumbler 1. The lower edge of the testing apparatus 4 is placed firmly against the skin 2 and moved from side to side such that the user can check to see if the rash 3 blanches or loses its colour under pressure. In addition, the user can now employ the reference section 8 to provide a direct comparison between the rash 3 on the skin 2 being tested and that associated with a particular disease being tested for.

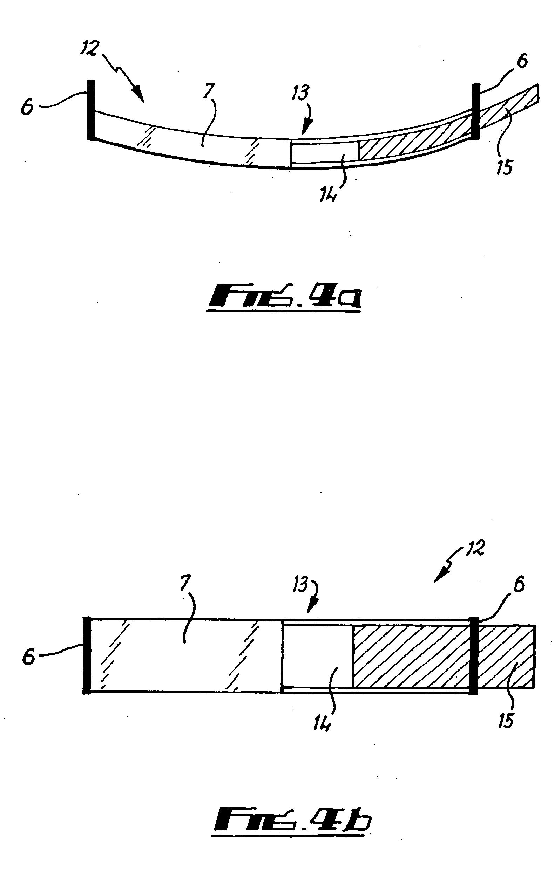

In an alternative embodiment 9 of the present inventions, shown in Figure 3, more than one reference section is employed within the testing apparatus. For example the first reference section 10 provides a representation of the rash associated with meningococcal meningitis while the second 11 provides a representation of the rash associated with septicaemia. This allows a user to test for both diseases simultaneously.

It will be obvious to one skilled in the art that the reference sections 10 and 11 may comprise any combination of known rashes with representations of meningococcal meningitis and septicaemia being provided by way of example only.

The aforementioned embodiments of the present invention are intended for use by members of the public as testing devices for skin rashes that are indicative of a disease that requires urgent medical attention. However, the testing apparatus may be readily adapted for use in the diagnosis of any distinctive skin rash, particularly by a medical practitioner.

Figure 4 presents a third embodiment 12 of the present invention ideally suited for such a purpose. This embodiment comprises a main body 13, in the form of a concave slide, and two handles 6. The main body 13 itself comprises a transparent viewing section 7 and a slot 14. The slot 14 is designed to enable the testing apparatus 12 to receive a reference cartridge 15, whereby the reference cartridge 15 provides the required representation of the skin rash to be tested for.

In this embodiment the slot 14 is designed so as to allow the reference cartridges 15 to be easily interchangeable. Therefore, by providing a series of reference cartridges 15, each one providing a representation of a different skin rash, the testing apparatus 12 is ideally suited for use by a medical practitioner in order to diagnose any disease associated with a characteristic skin rash.

Although the testing apparatus has been described as taking the form of a concave slid the invention is not restricted to such a shape. It is envisaged that a concave disc or any other suitable form may alternatively be employed.

Additionally, the testing apparatus could be further adapted such that the replication of the skin disease is provided via a computer and a LCD display rather than by the reference sections or cartridges as described above.

An advantage of the present invention is that it provides apparatus for testing human skin that aids the self diagnosis of a disease, where the disease is characterised by a particular skin rash. In particular it may be employed to diagnoses conditions that require urgent medical attention while reducing anxiety and stress induced by false self diagnosis that are indicative of the methods and apparatus described in the

### Prior Art.

A further advantage of the present invention is that it provides apparatus for testing human skin that aids the diagnosis of a disease by a medical practitioner, where the disease is characterised by a particular skin rash. By employing a series of interchangeable reference cartridges with the testing apparatus a number of diseases can be quickly tested for.

Although an exemplary embodiment of the present invention has been shown and described, it will be apparent to those having ordinary skill in the art that a number of changes, modifications or alterations to the invention described herein may be made, none of which depart from the invention as defined in the appended claims. All such changes, modifications, and alterations should therefore be seen as being within the scope of the present invention.

## Claims

1. Apparatus (4, 9, 12) for testing and diagnosing a rash present on human skin comprising a main body (5) wherein, the main body (5) comprises a transparent viewing section (7) and at least one reference section (8), each reference section (8) comprising at least one replication of a rash associated with a disease to be tested for
**characterised in that**
the main body (5) further comprises an arcuate surface (13) such that when the arcuate surface (13) is applied to the skin the apparatus (4) is free to oscillate.

2. Apparatus (12) as claimed in Claim 1 wherein the reference section (8) comprises a receiving means (14) and a removable cartridge (15).

3. Apparatus (12) as claimed in Claim 2 wherein the cartridge (15) comprises one or more replications of rashes associated with diseases to be tested for.

4. Apparatus (4, 9, 12) as claimed in any of the preceding claims wherein the replication of the rash comprises a representation of any rash associated with the human skin.

5. Apparatus (4, 9, 12) as claimed in any of the preceding claims wherein the replication of the rash comprises a representation of meningococcal meningitis.

6. Apparatus (4, 9, 12) as claimed in any of the preceding claims wherein the replication of the rash comprises a representation of septicaemia.

7. Apparatus (4, 9, 12) as claimed in any of the preceding claims wherein the apparatus further comprises one or more handles (6).

## Patentansprüche

1. Apparat (4, 9, 12) zum Untersuchen und Diagnostizieren eines auf der menschlichen Haut auftretenden Hautausschlags, umfassend ein Hauptkörperteil (5), wobei das Hauptkörperteil (5) einen durchsichtigen Beobachtungsabschnitt (7) und mindestens einen Bezugsabschnitt (8) umfasst, wobei jeder Bezugsabschnitt (8) mindestens eine Replikation eines mit einer Krankheit, auf die hin getestet werden soll, verbundenen Hautausschlags umfasst,
**dadurch gekennzeichnet, dass**
das Hauptkörperteil (5) des Weiteren eine gekrümmte Oberfläche (13) umfasst, derart, dass der Apparat (4), wenn die gekrümmte Oberfläche (13) auf die Haut aufgebracht wird, frei pendeln kann.

2. Apparat (12) nach Anspruch 1, wobei der Bezugsabschnitt (8) eine Aufnahmevorrichtung (14) und eine entfernbare Kartusche (15) umfasst.

3. Apparat (12) nach Anspruch 2, wobei die Kartusche (15) eine oder mehrere Replikationen von mit Krankheiten, auf die hin getestet werden soll, verbundenen Hautausschlägen umfasst.

4. Apparat (4, 9, 12) nach einem der vorhergehenden Ansprüche, wobei die Replikation des Hautausschlags eine Darstellung irgendeines mit der menschlichen Haut assoziierten Hautausschlags umfasst.

5. Apparat (4, 9 ,12) nach einem der vorhergehenden Ansprüche, wobei die Replikation des Hautausschlags eine Darstellung der Meningokokkenmeningitis umfasst.

6. Apparat (4, 9 ,12) nach einem der vorhergehenden Ansprüche, wobei die Replikation des Hautausschlags eine Darstellung von Septikämie umfasst.

7. Apparat (4, 9 ,12) nach einem der vorhergehenden Ansprüche, wobei der Apparat des Weiteren einen oder mehrere Griffe (6) umfasst.

## Revendications

1. Appareil (4, 9, 12) pour tester et diagnostiquer une éruption cutanée présente sur la peau humaine comprenant un corps principal (5) dans lequel le corps principal (5) comprend une section d'observation transparente (7) et au moins une section de référence (8), chaque section de référence (8) comprenant au moins une réplication d'une éruption cutanée associée à une maladie visée par le test ;
**caractérisé en ce que**
le corps principal (5) comprend en outre une surface courbe (13) de sorte que lorsque la surface courbe (13) est déposée contre la peau l'appareil (4) peut osciller.

2. Appareil (12) suivant la revendication 1 dans lequel la section de référence (8) comprend un moyen de réception (14) et une cartouche amovible (15).

3. Appareil (12) suivant la revendication 2 dans lequel la cartouche (15) comprend une ou plusieurs réplications d'éruptions cutanées associées à des maladies visées par le test.

4. Appareil (4, 9, 12) suivant l'une quelconque des revendications précédentes dans lequel la réplication de l'éruption cutanée comprend une représentation de n'importe quelle éruption cutanée associée à la peau humaine.

5. Appareil (4, 9, 12) suivant l'une quelconque des revendications précédentes dans lequel la réplication de l'éruption cutanée comprend une représentation de la méningite à méningocoques.

6. Appareil (4, 9, 12) suivant l'une quelconque des revendications précédentes dans lequel la réplication de l'éruption cutanée comprend une représentation d'une septicémie.

7. Appareil (4, 9, 12) suivant l'une quelconque des revendications précédentes dans lequel l'appareil comprend en outre une ou plusieurs poignées (6).
